# EUROPEAN PATENT APPLICATION

(11) **EP 4 282 460 A1**
(43) Date of publication of application: **29.11.2023**
(21) Application number: 22175158.9
(22) Date of filing: 24.05.2022
(51) Int. Cl.: A61M 25/10, A61B 90/00, A61M 25/04

(54) **A METHOD AND CATHETER CONTROL ASSEMBLEY FOR DETERMINING A FUNCTIONAL PARAMETER OF A CATHETER**

(71) Applicant: Wellspect AB, 431 21 Mölndal (SE)
(72) Inventor: D'Espindula, Gabriel, 418 71 Göteborg (SE); Eliasson, Göran, 444 94 Ucklum (SE)
(74) Representative: Lind Edlund Kenamets Intellectual Property AB

(57) **Abstract**

The present disclosure relates to a method and catheter control assembly for determining a functional parameter of a catheter. The method comprises connecting the catheter to a catheter connector interface comprising a first lumen configured to be in fluid communication with an inflatable retention element of the catheter and a second lumen configured to be in fluid communication with a main lumen of the catheter. The method further comprises injecting a fluid into the first lumen during a predetermined period of time, measuring a pressure parameter related to the pressure in the first lumen, and determining, based on the measured pressure parameter, a functional parameter of the catheter.

## Description

### TECHNICAL FIELD OF THE INVENTION

The present invention relates to a method and a catheter control assembly for determining a functional parameter of a connected catheter.

### BACKGROUND OF THE INVENTION

A catheter is a medical instrument useable for many types of uses and applications, comprising an elongated body through which one or more channels extends. By inserting a catheter in a bodily cavity or through the skin of a patient the channels of the catheter may be used to perform a wide range of medical procedures including enabling insertion of surgical instruments, drainage of fluids or administration of fluids through the one or more channels extending through the elongated body. The number and type of channels available in a catheter depends on the particular type of catheter and its intended use.

For instance, catheters may be used to perform rectal irrigation, such as trans anal irrigation (TAI), which involves introducing a catheter into the anal canal or rectum of a patient and injecting a fluid (e.g. water) to flush out stool and stimulate the bowels of the patient. Rectal catheters used for this purpose are often provided with a retention element, which may function to retain the catheter in an inserted position, and/or to form a seal against the anal canal when inserted into the rectum of a user. The retention element may e.g. be in the form of a cone which surrounds the elongated body and seals the anal canal or rectum when the catheter is used to inject fluid. Alternatively, a rectal catheter may be provided with an inflatable element (e.g. a balloon element) which surrounds the elongated body, and which is inflated inside the anal canal or rectum to create a seal and/or to retain the catheter in place. To this end, rectal catheters may feature at least two separate channels, a first channel for the injection of irrigation fluids and a second channel which at a first end is terminated inside the inflatable element for inflation of the inflatable element. By injecting a fluid, such as air or water, in the channel of the inflatable element the level of inflation may be controlled such that the inflatable element creates a seal and/or is inflated to allow the catheter to be retained in the inserted position. When the catheter is to be removed, the same channel may be used for deflation of the inflatable element.

A problem with prior solutions is that it could be difficult for medical professionals to keep track of the different types of catheters and also to separate used catheters from new catheters. Commonly a catheter is primed some time before use wherein the priming procedure may involve connecting the catheter to a catheter junction and e.g. flushing a fluid through one or more of the channels. In light of these problems, a solution has been proposed in WO20142185 involving attaching one or more magnets on the connector base of the catheter and using a Hall Effect sensor coupled to a controller in the catheter junction to detect the number or type of magnets on the catheter connector. By equipping different types of catheters with different numbers of magnets the controller of the catheter junction may determine the type of catheter based on measurements from the Hall Effect sensor and the controller may e.g. ensure that a proper procedure will be performed with the connected catheter or that correct information will be displayed for the medical professional.

A problem with this solution is however that existing catheters then need to be provided with a correct number of magnets to allow the Hall Effect sensor of the catheter junction to properly detect the type of catheter and that the system is incapable of determining if a catheter has been previously used. This known solution is complicated and costly, and cumbersome to use.

### SUMMARY OF THE INVENTION

In view of the drawbacks of existing solutions there is a need for an improved method and catheter arrangement capable of determining the type of catheter. It is a purpose of the present invention to provide such an improved method and catheter arrangement which facilitates determination of a functional parameter of a catheter.

According to a first aspect of the invention there is provided a method for determining a functional parameter of a catheter wherein the method comprises connecting the catheter to a catheter connector interface wherein the catheter connector interface comprises a first lumen and a second lumen. The first lumen is configured to be in fluid communication with an inflatable retention element of the catheter and the second lumen is configured to be in fluid communication with a main lumen of the catheter. The method further comprises the steps of injecting a fluid into the first lumen during a predetermined period of time, measuring a pressure parameter related to the pressure in the first lumen and determining a functional parameter of the catheter based on the measured pressure parameter.

With a fluid it is meant any liquid, gas or material which flows (such as a foam) or combination thereof. In embodiments, the fluid is a gas, such as ambient air, or a liquid, such as an irrigation liquid, e.g. water or saline.

With functional parameter it is meant a parameter indicating a property of the catheter. The property may be one or more properties selected from a group comprising: (a) whether the catheter comprises an inflatable retention element, (b) whether the inflatable retention element of the catheter has been used (wherein used means that the inflatable retention element has been inflated previously) previously, (c) whether the inflatable retention element of the catheter is new (wherein new means that the inflatable retention element has not been inflated previously) and (d) the size/volume of the inflatable retention element. Determining the size/volume of the inflatable retention element may comprise determining the absolute size/volume of the inflatable retention element or determining if the inflatable retention element is of a first size or second size. Determining the size/volume of the inflatable retention element may comprise determining which size/volume out of at least two sizes/volumes that corresponds to the inflatable retention element. The catheter may be any type of catheter, preferably the catheter is a rectal irrigation catheter, a urinary tract catheter or a ureteric balloon catheter.

The inflatable retention element may be a balloon element made from an elastic or resilient material.

The present invention is at least partially based on the understanding that the pressure parameter depends on the functional parameter of the catheter. Therefore, by injecting a fluid and measuring the pressure parameter an analysis of the measured pressure parameter will yield the functional parameter without necessitating a new type of catheter or retrofitting existing catheters with e.g. magnets that can be detected with Hall Effect sensors.

In an embodiment, the catheter control assembly is arranged to determine whether a catheter connected to the assembly comprises an inflatable retention element or not. In an embodiment, the catheter control assembly is arranged to determine whether the inflatable element of a catheter connected to the assembly has been previously inflated or not. In an embodiment, the catheter control assembly is arranged to determine a size or particular type of a catheter connected to the assembly.

It is of advantage to be able to ensure that medical professionals, or patients performing self-treatment, use a catheter that is intended for the treatment which is to be performed. Hereby, it may be ensured that the catheter control assembly is not used with a catheter for which it is not suited, or that the catheter control assembly is controlled in a way unsuitable for the catheter. Additionally, or alternatively, for single use catheters it may be of advantage that medical professionals, or patients performing self-treatment, do not use a catheter that has been used previously. A used catheter may have properties that have been impaired by the previous use, may be contaminated, or may for other reasons be unsuitable for a repeated, subsequent use.

With the method of the present invention the functional parameter of a catheter may be automatically determined when the catheter is connected to the catheter connector interface, e.g. during a priming of the catheter. The functional parameter may then be presented to the medical professional, or patient performing self-treatment, so as to e.g. issue a warning if a previously used catheter has been connected to the catheter connector interface. Additionally or alternatively, the functional parameter may be used to control the treatment procedure, e.g. if the functional parameter indicates that the catheter does not comprise an inflatable retention element a valve or pressure source may be controlled to ensure that no fluid is injected to the first lumen during the treatment procedure.

In some implementations, the pressure parameter is based on a fluid pressure and the step of measuring a pressure parameter related to the pressure in the first lumen further comprises performing at least one measurement the fluid pressure in the first lumen with a pressure sensor.

When a fluid is injected into the first lumen the pressure in first lumen will change in a manner which depends on the functional parameter of the catheter. By measuring the pressure in the first lumen the functional parameter of the catheter may be determined based on the measured pressure. The pressure parameter may e.g. be based on a direct measurement of the fluid pressure, an average fluid pressure, a maximum fluid pressure, or a rate of change (derivative) of the pressure during a predetermined measurement period of time in the first lumen. For example, the fluid pressure is measured with a pressure sensor. The pressure sensor is for example a pressure transducer.

Additionally or alternatively, the pressure parameter is based on an indirect measurement of the fluid pressure in the first lumen. If an electric pump is used to pump the fluid into the first lumen the voltage or current (or power) supplied to the electric pump will indirectly indicate the fluid pressure in the first lumen as the work performed by the electrical pump will vary over time depending on the functional parameter of the connected catheter. Accordingly, the pressure parameter may be based on a measurement of at least one of the current, voltage and power supplied to the electric pump which indirectly indicate the pressure in the first lumen. Specifically, the pressure parameter may be a particular measurement sample of the pump voltage or current, an average pump voltage or current, a maximum pump voltage or current, a rate of change (derivative) of the pump voltage or current during a predetermined measurement period of time, all of which indirectly indicates the pressure in the first lumen.

In some implementations, determining a functional parameter comprises determining if the pressure parameter is above a first predetermined threshold. If it is determined that the pressure parameter is above the first predetermined threshold the functional parameter is assigned with a first value, otherwise the functional parameter is assigned with a second value.

Accordingly, the functional parameter may be determined by analyzing whether the pressure parameter exceeds the predetermined threshold. By measuring the pressure parameter for reference catheters with different and known functional parameters the predetermined threshold may be established and then used to determine the functional parameter of catheters connected to the catheter connector interface.

More than one predetermined threshold may be provided, e.g. determining a functional parameter may further comprise determining if the pressure parameter is above a second predetermined threshold value wherein the second predetermined threshold value is higher than the first predetermined threshold value. If the pressure parameter is above the second predetermined threshold the functional parameter is assigned with a third value.

In some implementations, the first value of the functional parameter indicates that the catheter comprises a new inflatable retention element and wherein the second value of the functional parameter indicates that the catheter comprises a used inflatable retention element. As will be described in the below, a catheter comprising an inflatable retention element which has not been used previously may be expected to be associated with a pressure parameter that is higher than a pressure parameter of a catheter with a previously used inflatable retention element. Additionally, the third value of the functional parameter may indicate that the catheter is not provided with an inflatable retention element. A catheter without an inflatable retention element may feature a sealed inflation lumen, or no inflation lumen, meaning that the first lumen of the catheter connector interface is not in fluid communication with a volume which changes substantially when a fluid is injected. This means that the pressure parameter may increase continuously or stabilize at higher levels in comparison to catheters which does comprise an inflatable retention element. Thus, the second threshold may be selected to separate such catheters from catheters with any form of inflatable retention element.

In some implementations, measuring a pressure parameter related to the pressure in the first lumen comprises measuring a plurality of pressure parameter samples, wherein the pressure parameter is based on the plurality of pressure parameter samples. For example, the pressure parameter may be based on the maximum pressure parameter sample or a difference between at least two pressure parameter samples of the plurality of pressure parameter samples.

The pressure parameter samples describe how the pressure parameter varies over time, i.e. defining a pressure parameter function. Accordingly, the pressure parameter function will depend on the functional parameter of the catheter and by analyzing the pressure parameter function (comprising at least two pressure parameter samples) the functional parameter of the catheter may be determined.

Determining the pressure parameter may comprise comparing a sequence of pressure parameter samples (e.g. a pressure parameter function) with a database of sequences of pressure parameter samples obtained for catheters with different functional parameters and determining the closest matching sequence of pressure parameter samples. The associated functional parameter of the closest matching sequence may be the determined functional parameter. It is noted that many alternatives exist for determining the functional parameter of a pressure parameter given reference measurements of the pressure parameter for characters with known functional parameters.

In some implementations, the first lumen is separated from a pressure source with one or more valves and the method further comprises opening at least one valve for the predetermined period of time to inject a pressurized fluid into the first lumen from the pressure source during the predetermined period of time.

Alternatively, the first lumen may be in fluid communication with a pump and the method may further comprise activating the pump for the predetermined period of time to inject a pressurized fluid into the first lumen using the pump during the predetermined period of time. Accordingly, the fluid may be injected to the first lumen for a suitable predetermined period of time which may be adjusted by controlling the valve and/or pump.

In some implementations, measuring the pressure parameter comprises measuring the pressure parameter during the predetermined period of time. Accordingly, the pressure parameter may be measured when the fluid is injected to the first lumen.

Additionally or alternatively, the predetermined period of time is a first period of time, wherein the method further comprises ceasing injection of the fluid after the predetermined first period of time. Wherein measuring the pressure parameter comprises measuring the pressure parameter during a second period of time, the second period of time being subsequent to the first period of time. Accordingly, the pressure parameter may be measured after the fluid has been injected. Optionally, the method comprises opening a venting valve for venting the fluid from the first lumen. For instance, the venting valve is opened during the predetermined second period of time.

In some implementations, the predetermined period of time is between 1 millisecond and 10 seconds and preferably between 3 milliseconds and 5 seconds. The pressure parameter is influenced by the functional parameter from the start of the fluid injection. Accordingly, a short predetermined period of time of a few milliseconds may be sufficient to determine the functional parameter of the catheter. Alternatively, a long predetermined period of time of at least 0.5 seconds may be used to determine the functional parameter.

According to a second aspect of the invention there is provided a catheter control assembly comprising a catheter connector interface, wherein the catheter connector interface comprises a first lumen configured to be in fluid communication with an inflatable retention element of the catheter, and a second lumen configured to be in fluid communication with a main lumen of a catheter. The catheter control assembly further comprises a catheter controlling unit, the catheter controlling unit being configured to operate fluid injection means to inject a fluid into the first lumen during a predetermined period of time, measure a pressure parameter related to the pressure in the first lumen, and determine, based on the measured pressure parameter, a functional parameter of the catheter.

For instance, the catheter controlling unit may comprise a controller and a sensor for operating the fluid injection means and measuring the pressure parameter respectively.

The catheter control assembly may further comprise a pump, and preferably an electric pump. The pump may be arranged integrated within a single housing of the catheter control assembly. However, alternatively, the pump may be provided as a separate, external pump. The catheter control assembly may further comprise a valve, separating an output of the pump from the catheter connector interface. Additional components may also be provided in the catheter control assembly, such as sensors, an energy source, such as a battery, additional pumps, etc. A reservoir for accommodation of a supply of irrigation liquid may also be provided, arranged integrated or connected to the assembly.

The controlling unit may be realized in software executed in a processor, such as a CPU, a microcontroller, etc. The controlling unit may also be realized partly or fully in hardware. The controlling unit is preferably arranged as an integrated part of the catheter control assembly, and preferably arranged within a housing thereof. However, alternatively, the controlling unit may be arranged externally, and connected to the rest of the assembly through a wired or wireless communication connection.

The fluid injection means may comprise a pump and/or a valve, wherein the valve is configured to control the flow from a vessel containing a pressurized fluid.

According to a third aspect of the invention there is provided a computer program product comprising instructions which, when executed by a computer, performs the method according to the first aspect of the invention. The computer program product, such as computer executable software, may be provided on a data carrying article of manufacture, such as a data storage.

The invention according to the second and third aspect features the same or equivalent benefits as the invention according to the first aspect. Any functions described in relation to a method may have corresponding features in a device or arrangement, and vice versa.

### BRIEF DESCRIPTION OF THE DRAWINGS

The present invention will be described in more detail with reference to the appended drawings, showing currently preferred embodiments of the invention.
Figure 1 depicts a block diagram of a catheter arrangement according to some embodiments of the present invention.
Figure 2 illustrates schematically, in cross-section, a catheter with an inflatable retention element according to some embodiments of the present invention.
Figure 3 illustrates schematically, in cross-section, a catheter arrangement connected to a catheter with an inflatable retention element according to some embodiments of the present invention.
Figure 4 is a flowchart illustrating a method according to some embodiments of the present invention.
Figure 5a illustrates a control signal controlling a pressure source or a valve, as a function of time, according to some implementations of the invention.
Figure 5b illustrates the pressure inside the first lumen as a function of time when a fluid is injected into the first lumen for connected catheters with different functional parameters.
Figure 5c illustrates the current or voltage supplied to a pump which is injecting a fluid into the first lumen for connected catheters with different functional parameters.
Figure 6a illustrates the current or voltage supplied to a pump which is injecting a fluid into catheters with new inflatable retention elements of different sizes.
Figure 6b illustrates the current or voltage supplied to a pump which is injecting a fluid into catheters with used inflatable retention elements of different sizes.
Figure 7a illustrates the pressure inside the first lumen when a fluid is injected for connected catheters with new inflatable retention elements of different sizes.
Figure 7b illustrates the pressure inside the first lumen when a fluid is injected for connected catheters with used inflatable retention elements of different sizes.
Figure 8a illustrates a short control signal controlling a pressure source or a valve, as a function of time, according to some implementations of the invention.
Figure 8b illustrates the pressure inside the first lumen when a fluid is injected, as a function of time, for connected catheters with different functional parameters.

### DETAILED DESCRIPTION OF CURRENTLY PREFERRED EMBODIMENTS

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the medical device, etc. Further, even though the discussed embodiments are in particular suitable for use in rectal irrigation assemblies, i.e. catheter control assemblies to be connected to rectal catheters, e.g. for use in trans anal irrigation, similar catheter control assemblies may also be used for other applications, such as for dilation catheters, venous catheters, urinary catheters, and the like.

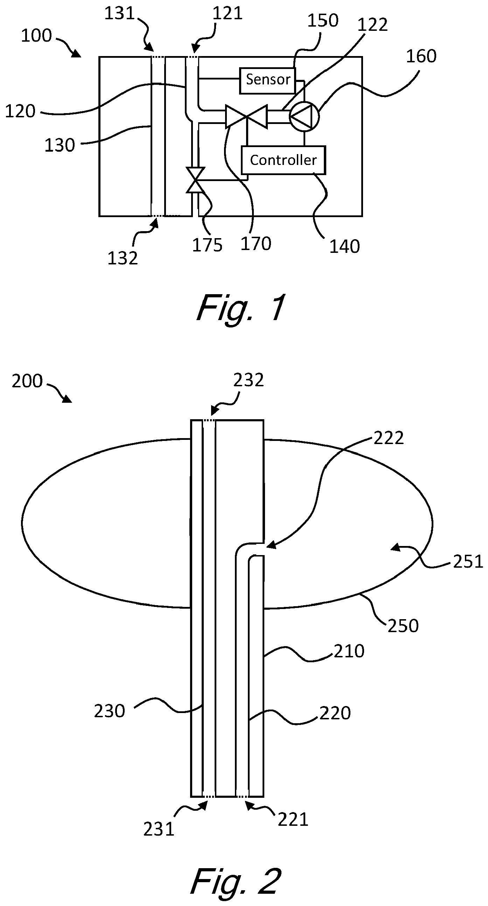

Fig. 1 depicts a block diagram of a catheter control assembly 100 comprising a second (passthrough) lumen 130 and a first lumen 120 wherein the second lumen 130 is configured to be in fluid communication with a main lumen of a catheter via a catheter connection interface through a catheter connection port 131 and the first lumen is configured to be in fluid communication with an inflatable retention element of the catheter via the catheter connection interface through an inflation port 121. The illustrated catheter control assembly 100 comprises two lumens, a first lumen 120 and a second lumen 130, but may comprise additional lumens in any suitable arrangement. For instance, the catheter control assembly 100 may comprise at least two second (passthrough) lumens 130 in addition to the first lumen 120 and the first lumen 120 and second lumen(s) 130 may be arranged concentrically as opposed to the side-by-side arrangement of the embodiment shown in fig. 1.

The second lumen 130 extends between the first catheter connection port 131 and the first passthrough port 132 to enable e.g. a fluid or a surgical instrument to be passed through the second lumen 130 of the catheter control assembly 100. Although the catheter control assembly 100 in fig. 1 is depicted as single unit the catheter control assembly 100 may comprise separate units. For instance, a catheter connector interface comprising the second lumen 130 and the first lumen 120 may be provided as at least one separate unit(s) while the controller 140 and sensor 150 are provided as another separate unit (a catheter controlling unit) and the pressure source 160 is provided as yet another separate unit.

The catheter control assembly 100 preferably comprises a pressure source 160 in fluid communication with the first lumen 120. The pressure source 160 may be a pressure vessel containing a fluid under pressure and/or a pump configured to pump a fluid into the first lumen 120. The pump may be any suitable pump such as a mechanical pump, electric pump or electromechanical pump. For example, the pump may be a positive displacement pump, a rotary positive displacement pump (e.g. a screw pump or gear pump), a reciprocating pump (e.g. a plunger/piston pump or a diaphragm pump), a centrifugal pump or a peristaltic pump. Preferably, the pump is an electric pump. Different type of pumps may exhibit different voltage and/or current characteristics as a fluid is injected into the first lumen 120 but a difference in the voltage and/or current functions can be observed for different catheters with any type of pump provided sufficient voltage and/or current measurement accuracy. Accordingly, the pump voltage and/or current may be used as a pressure parameter for any type of electric pump.

Another pressure source or a medical instrument may be connected to/passed through the passthrough port 132 to e.g. inject or remove a fluid to the catheter when performing treatment.

The pressure source 160 may be associated with a valve 170 which separates the pressure source 160 from the first lumen 120. In some implementations, the pressure source 160 is a pressure vessel and the valve 170 is a valve of the pressure vessel, accordingly the valve 170 and a pressure vessel may together form a controllable pressure source. In some implementations, there is a buffer volume 122 between the pressure source 160 and the valve 170. The valve 170 may be three-way valve wherein the valve 170 is further configured to vent the first lumen 120. Alternatively, a venting valve 175 is provided which is configured to vent the first lumen 120 after the predetermined period of time. The venting valve 175 may further be configured to be closed during the predetermined period of time so as to retain the injected fluid in the first lumen 120. The venting valve 175 may be controlled by the controller 140.

The catheter control assembly 100 may further comprise a sensor 150 configured to sense a pressure parameter related to the pressure in the first lumen 120. For instance, the sensor 150 may be a pressure sensor configured to measure the pressure in the first lumen 120. Alternatively, or additionally, the sensor 150 may be a voltage, current or power sensor configured to measure the pressure indirectly, through a voltage, current or power supplied to the pressure source 170 wherein the pressure source e.g. is an electrical pump.

Moreover, the catheter control assembly 100 may comprise a controller 140 for controlling at least one of the pressure source 160 and the valve 170. The controller 140 may be configured to activate the pump for a predetermined period of time and optionally inactivate the pump after the predetermined period of time has elapsed. The controller 140 may be configured to open the valve 170 for a predetermined period of time and close the valve 170 after the predetermined amount of time has elapsed. If the pressure source 160 is a pressure vessel the valve 170 may be the valve of the pressure vessel.

Specifically, it is noted that a pump and a valve 170 may be combined wherein the controller 140 may be configured to activate the pump prior to, at the same time as or after the valve 170 has been opened. Additionally, or alternatively, the controller 170 may be configured to inactivate the pump prior to, at the same time as or after the valve has been closed. Thus, the controller 140 may e.g. be configured to enable a resting period of time during which the valve 170 is open and the pump is inactive.

The controller 140 may control the sensor 150 to perform measurements at suitable times. The controller 140 may further be configured to periodically update the functional parameter by periodically injecting a fluid into the first lumen 120 and controlling one or more sensors 150 to measure the pressure parameter related to the fluid pressure in the first lumen 120. The controller may also be configured to control the one or more valves 170.

Fig. 2 illustrates schematically a catheter 200 comprising an elongated body 210 through which at least two lumens 220, 230 extend at least partially. The catheter may comprise a catheter connector, connectable to the catheter connector interface of the catheter control assembly. In the depicted embodiment a main lumen 230 extends from a first connector port 231 of a first end of the elongated body 210 to a second port 232 of a second end of the elongated body 210. When in use, the main lumen 230 of the catheter 200 may be used to introduce or remove a surgical instrument or a fluid to/from the patient via the first connector port 231 and the second port 232. The catheter 200 may comprise more than one main lumen 230 extending from substantially the first end to the second end of the catheter 200 such as two, three or more main lumens 230. Multiple main lumens 230 may be provided to enable simultaneous use of an instrument, administering of at least one fluid and/or removal of at least one fluid (using e.g. suction or a negative pressure at the ports 231 of the first end of the catheter 200).

The catheter 200 in fig. 2 further comprises an inflatable retention element 250 fixated to the elongated body 210 and at least partially surrounding the elongated body 210. The inflatable retention element 250 may be of different sizes depending on the intended use of the catheter 200 and similarly the inflatable retention element 250 may be made of any suitable flexible or elastic material, such as a plastic material. The inflatable retention element 250 forms an inflatable volume 251 which is in fluid communication with the inflation lumen 220 via a second inflation port 222. The inflation lumen 220 extends to a first inflation port 221 on the first end of the catheter 200. By injecting a fluid into the inflation lumen 220 via the first inflation port 221 the inflatable volume 251 may be increased and by e.g. providing a suction or venting of the inflation lumen 220 the volume 251 of the inflatable retention element 250 may be decreased. Accordingly, by inserting the elongated body 210 into e.g. the anal canal or rectum of the patient, with the inflatable retention element 250 in a deflated state, the inflatable retention element 250 may be inflated by injecting a fluid into the inflation lumen 220, such as water or air, whereby the inflatable retention element 250 inflates to retain the catheter in the inserted position, and may additionally form a seal against the body of the patient for retaining fluids inside the patient, such as fluids injected via the second port 232 of the main lumen 230 inside the patient.

It is further noted that while the main lumen(s) 230 and the inflation lumen 220 are depicted as separate lumens being arranged side-by-side in the catheter 200 other channel layouts are possible, e.g. the main lumen 230 may be concentrically placed inside inflation lumen 220 or vice versa.

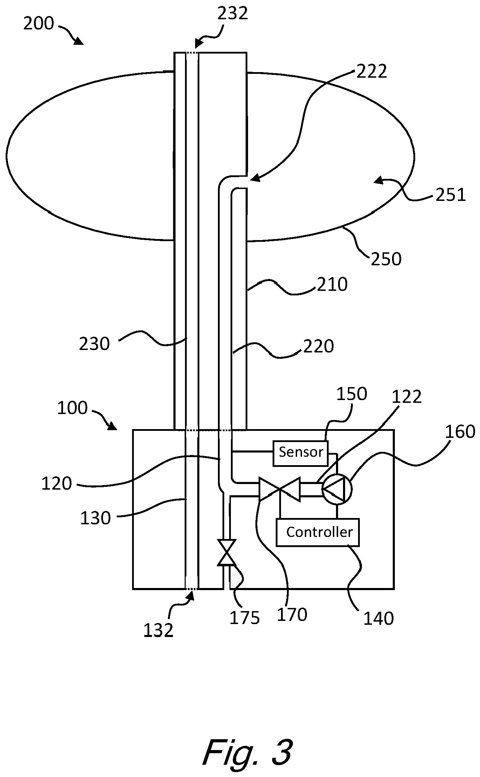

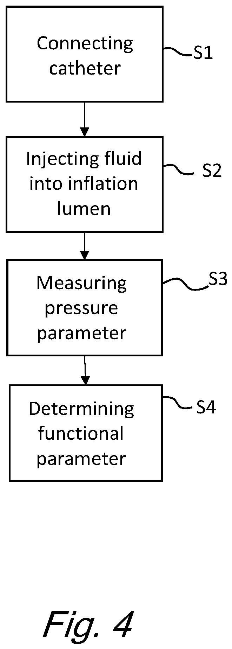

Fig. 3 illustrates the catheter control assembly 100 being connected to the catheter 200. It is noted that while the catheter control assembly 100 is depicted as being directly connected to the catheter 200 the catheter control assembly 100 may be connected to the corresponding lumens of the catheter 200 via one or more extension tubes (not shown). With further reference to Fig. 4 a method for determining a functional parameter of the catheter 200 will now be described.

At step S1 a catheter 200 is connected to a catheter connector interface comprising a second lumen 130 configured to be in fluid communication with a main lumen 230 of the catheter 200 and a first lumen 120 configured to be in fluid communication with an inflatable retention element 250 of the catheter 200. If the catheter 200 does not comprise an inflatable retention element 250 the first lumen 120 of the catheter connector interface configured to be in fluid communication with an inflatable retention element 250 of the catheter 200 may be in fluid connection with a lumen of the catheter 200 which is closed, i.e. is not in fluid communication with an inflatable retention element 250 or the environment of the catheter 200. As a further example, the catheter 200 may lack the inflation lumen 220 altogether whereby the first lumen 120 of the catheter arrangement 100 is closed at the inflation port 121 when the catheter 200 is connected to the catheter connector interface. Alternatively, the second inflation port 222 may be closed meaning that the second lumen 120 is in fluid communication with the substantially fixed volume of the inflation lumen 220.

Some catheters used for rectal irrigation does not comprise an inflatable retention element which is in fluid communication with the first lumen 220 and instead comprises no retention elements at all, or other types of retention elements, such as a cone element pointing towards the second end of the catheter 200 (comprising the second port 232) and provided around the elongated body 210. The cone element is used as an alternative method for retaining the catheter in place and creating a seal against the patient's body. As an alternative to a cone element the catheter may comprise a closed (or static) fluid filled volume (e.g. a ball shaped element made from a non-elastic material) which is at least partially filled with a fluid when the user inserts the catheter, uses the catheter for irrigation and removes the catheter. While this closed fluid filled volume in some sense is an inflatable retention element it is not intended to be inflated after the user has inserted the catheter and it would not be in fluid communication with the first lumen 220. Accordingly, the pressure parameter will indicate that the first lumen 220 is not in fluid communication with an inflatable retention element when a closed (or static) fluid filled volume is attached to the catheter to facilitate proper sealing during use as the closed (or static) fluid filled volume is isolated from the first lumen 220.

It is noted that some catheters may comprise an inflatable retention element 250, a cone element or both.

At step S2 a fluid is injected into the first lumen 120 of the catheter arrangement 100 which may result in the fluid also being injected into the inflation lumen 220 (if present in the catheter 200) and injected into the inflatable retention element 250. For instance, the controller 140 of the catheter arrangement 100 may perform at least one of activating a pump and opening the valve 170 whereby a fluid is injected into the first lumen 120.

The method proceeds to step S3 comprising measuring the pressure parameter. Step S3 may comprise at least one of measuring with the sensor 150 the pressure of the first lumen 120 and measuring the current, voltage or power supplied to the pump. The measurements) of the pressure parameter acquired by the sensor 150 may be provided to a processing unit, such as the controller 140, configured to determine, based on the measurements), a functional parameter of the catheter 200. Accordingly, the method may go to step S4 comprising determining functional parameter of the catheter 200 based on the measured pressure parameter.

In some implementations, the sensor 150 performs at least one measurement of the pressure parameter as the valve 170 is open and/or the pressure source 160 is active. Additionally, or alternatively, the controller 140 may be configured to close the valve 170 or inactivate the pump 160 whereby the sensor 150 performs at least one measurement of the pressure parameter as the valve 170 is closed and/or the pressure source 160 is inactive. Optionally, a venting valve 175 is opened prior to, during or after the valve 170 is closed and/or the pressure source 160 is inactive. In a further example, the controller 140 may inactivate the pressure source 160 while the venting valve 175 is closed wherein the sensor performs at least one measurement of the pressure parameter when the pressure source 160 is inactive (during a predetermined resting time).

In some implementations, the at least one measurement is taken when the pressure in the first lumen 220 has stabilized. Alternatively, the at least one measurement is taken at a predetermined time after the valve 170 has started to open, or has become fully open, while the pressure inside the first lumen 220 is still changing. Regardless of when the at least one measurement of the pressure parameter is taken during pressurization of the first lumen 220, it will be indicative of the functional parameter of the catheter. For instance, after a predetermined time since the valve 170 was opened the pressure in the first lumen 220 may be a first value if the first lumen 220 is not connected to an inflatable element, a second value if the first lumen 220 is connected to a not previously used inflatable element and a third value if the first lumen 220 is connected to a previously inflated inflatable element wherein the first value is greater than the second value and the second value is greater than the third value.

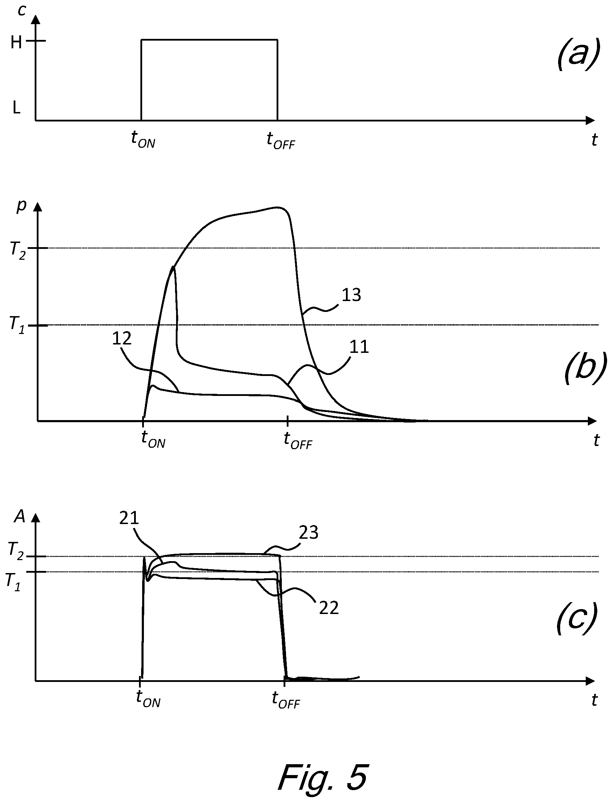

Fig. 5a depicts a signal c as a function of time representing the predetermined period of time and/or a control signal outputted by the controller. With further reference to fig. 3 the control signal c may be a control command passed from the controller 140 to the valve 170 and/or pressure source 160 (pump) wherein the high level H of the control signal c indicates when e.g. a pump is activated or when the valve is opened to inject a fluid under pressure into the first lumen 120. That is, the high level H of the control signal c indicates the extent of the predetermined period of time. Similarly, the low level L of the control signal c indicates that the pump is inactivated or that the valve 170 is closed. The control signal c assumes the high value for a predetermined period of time between time t_{ON} and time t_{OFF} during which a fluid is injected to the first lumen 120. The high level H of the control signal c may also indicate that the venting valve 175 is closed and the low level L may indicate that the venting valve 175 is opened.

In some implementations, the pressure source 160 comprises a pump and it is envisaged that the pump may be active independently of the control signal (e.g. constantly active or active for a duration much longer than the duration of the high level H of the control signal). Alternatively, the pump may be activated when the control signal is in the high level. For example, the valve 170 opens at the same time the pump is activated. A benefit with having the pump already activated when the valve 170 is opened is that a valve may be opened very rapidly which means that the first lumen 220 becomes pressurized rapidly.

With further reference to fig. 5b there is depicted the fluid pressure inside the first lumen 120 as functions 11, 12, 13 of time for three connected catheters 200 with different functional parameters. Namely a pressure function 13 for a catheter without an inflatable element, a pressure function 11 for catheter with an inflatable element which has not been used and a pressure function 12 for a catheter which has been used previously. As seen, from t_{ON} the pressure inside the first lumen 120 starts to increase for all catheters (albeit at different rates). The maximum pressure reached during the predetermined period of time depends on whether the catheter 200 comprises an inflatable retention element 250, the size of the inflatable retention element 250 and whether the inflatable retention element 250 has been used before.

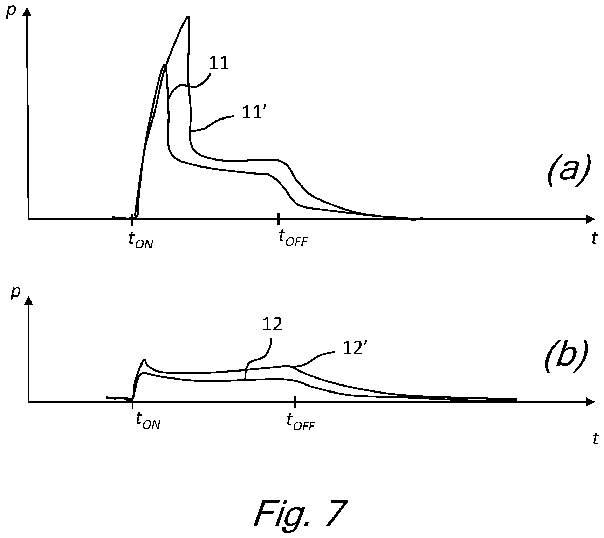

The pressure functions 11, 12, 13 in Fig. 5b illustrates approximately the pressure inside the first lumen 120 as functions of time for different catheters wherein the used or new inflatable element is of the same size (when present). A comparison of the pressure as a function of time for catheter with different sizes of the inflatable retention element 250 is described in connection to fig. 7a and 7b in the below.

Accordingly, a first threshold T₁ may be established so as to separate the maximum pressure reached for a catheter with a used inflatable retention element 250 (pressure function 12) from the maximum pressure reached for a catheter with a new (not previously inflated) inflatable retention element 250 (pressure function 11). As the expected maximum pressure for a catheter with a new inflatable retention element 250 is higher than the expected maximum pressure obtained for a catheter with a used inflatable retention element 250 the threshold T₁ may be at least higher than the expected maximum pressure reached for a catheter with a used inflatable retention element.

Additionally or alternatively a second threshold T₂ may be established so as to separate the maximum pressure reached for a catheter with a new (not previously inflated) inflatable retention element 250 (pressure function 11) from a catheter 200 which does not comprise an inflatable retention element 250 (pressure function 13). As indicated in the above, a catheter 200 without an inflatable retention element 250 may provide the first lumen 120 with a fixed volume which cannot be inflated. Accordingly, the fluid pressure will continue to increase during the predetermined period of time in contrast to the pressure for catheters 200 with an inflatable retention element 250 that may exhibit the characteristic pressure peak preceding a pressure decrease.

Similarly, the rate of change (derivative) for the fluid pressure differs between the measured pressure functions 11, 12, 13. Especially for the onset rate after t_{ON} or the comparatively stable pressure reached after the initial peak for the pressure functions 11, 12. Accordingly, at least one rate of change (derivative) threshold may be established which separates the three types of catheters based on the rate of change of the pressure during different intervals of the predetermined period of time. Additionally, the rate of pressure decrease due to deflation or venting using the venting valve 175 after t_{OFF} also varies depending on the type of catheter and thereby at least one rate of change threshold may be established so as to separate the three types of catheters based on the measured pressure after t_{OFF}.

Additionally, fig. 5c illustrates the current supplied to the pump as a function of time for the three catheters with different functional parameters. Current function 23 is for a catheter without an inflatable element, current function 21 is for a catheter with an inflatable element which has not been used and current function 22 is for a catheter which has been used previously.

A constant voltage source was connected to the pressure source 160 in the form of an electrical pump when measuring the current functions 21, 22, 23. However, the voltage functions of the pump may exhibit similar characteristics to the current functions 21, 22, 23 if a constant current source where to be employed instead of constant voltage source.

As seen, the current rapidly increases to a peak value for each catheter whereby the current 21, 22, 23 subsequently fluctuates towards a steady-state value. The steady state current differs if the pump is pumping the fluid to a catheter without an inflatable element (current function 23), to a catheter which has an inflatable retention element 250 that is inflated for the first time (current function 21) or if the inflatable retention element 250 has been inflated before (current function 22). Similarly, a local maximum value of the current is reached for catheters with a (new or used) inflatable element 250 wherein the local maximum current and the timing of the local maximum with respect to t_{ON} differs depending on if the inflatable retention element 250 is new or has been inflated before. Moreover, the local maxima correspond in time approximately with pressure peaks of the corresponding pressure functions 11, 12, 13 from fig. 5b.

Accordingly, by analyzing the steady state current, the timing of the local maximum with respect to t_{ON} or the local maximum current the functional parameter of the catheter may be determined. For instance, a threshold T₁ may be established so as to separate a catheter with a new inflatable retention element from a catheter with a used inflatable retention element. For instance, the threshold T₁ may be above the steady state current associated with a pump pumping a fluid into a catheter with a used inflatable retention element 250 (current curve 22) but below the steady state current associated with a pump pumping a fluid into a catheter with a new inflatable retention element 250 (current curve 21). Additionally or alternatively, a second threshold T₂ may be established so as to separate a catheter 200 without an inflatable retention element 250 (current curve 23) from a catheter 200 with a used inflatable retention element 250 (current curve 22) or new inflatable retention element (current curve 21). For instance, the threshold T₂ for determining if no inflatable intention element is present may be above the steady state current associated with a pump pumping a fluid into a catheter 200 with a new inflatable retention element 250 (current curves 21) but below the steady state current associated with a pump pumping a fluid into a catheter 200 without an inflatable retention element 250 (current curve 23).

As a further example, it may be determined that a new inflatable retention element 250 is present if the current is between the two thresholds T₁ and T₂.

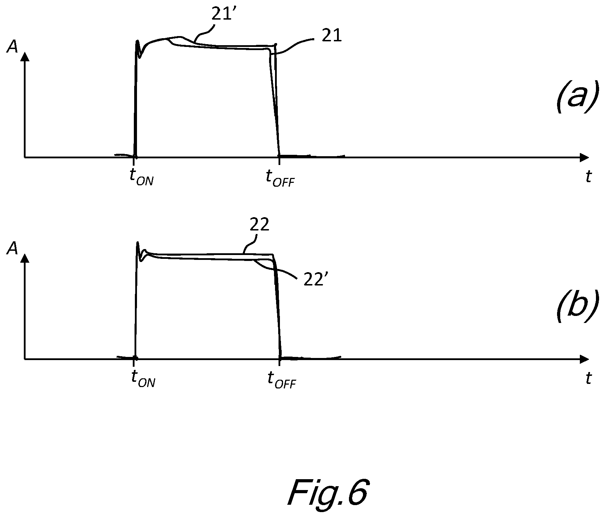

Fig. 6a and 6b illustrates how the current supplied to the pump changes over time between t_{ON} and t_{OFF} for connected catheters 200 with inflatable retention elements 250 of different sizes. In fig. 6a the current functions 21, 21' illustrates the current supplied to the pump as a function of time when the catheter comprises a new inflatable retention element 250 of a first size/volume (current function 21) and a new inflatable retention element 250 of a second size/volume (current function 21') wherein the second size is smaller than the first size. Analogously, fig. 6b depicts current functions 22, 22' illustrating the current supplied to the pump as a function of time when the catheter comprises a used inflatable retention element 250 of a first size/volume (current function 22) and a used inflatable retention element 250 of a second size/volume (current function 22') wherein the second size is smaller than the first size.

As seen, the steady state current, the local peak current value following t_{ON}, and the duration of the local peak differs depending on the size of the new or used inflatable retention element 250. Accordingly, at least one predetermined threshold may be determined so as to identify whether the inflatable retention element 250 is of the first or second size based on a measurement of the current supplied to the pump.

Similarly, the measured pressure as a function of time may be used to determine the size of the catheter. With reference to fig. 7a there is depicted the pressure as a function of time for a catheter with a new inflatable retention element 250 of a first size/volume (pressure function 11) and of a second size/volume (pressure function 11') wherein the second size/volume is smaller than the first size. Accordingly, by determining e.g. the peak value, rate of change or shape of the pressure function 11, 11' the size of the inflatable retention element 250 may be determined. With reference to fig. 7b there is illustrated approximate pressure curves 12, 12' for the fluid pressure in the first lumen 120 for a catheter with a used inflatable retention element 250 of a first size/volume (pressure function 12) and a used inflatable retention element 250 of a second size/volume (pressure function 12') wherein the second size/volume is smaller than the first size. Accordingly, the differences between the pressure curves 12, 12' may be used to determine the size of the used inflatable retention element 250.

The predetermined period of time between t_{ON} and t_{OFF} in fig. 5, 6 and 7 may be referred to as a long period of time or a long pulse. With a long pulse it is meant a pulse which is 0.3 seconds or longer such as a pulse which is at least 1 or 2 seconds long to enable the inflatable retention element to inflate sufficiently such that a characteristic peak is reached for the fluid pressure in the first lumen or the voltage and/or current supplied to the pump. While approximately 0.3 seconds was sufficient for the pressure source used in experiments to ensure that the characteristic peak occurs the required time may be shorter or longer depending on the length of the catheter 200, the size and material of the inflatable retention element 250, the pressure of the pressure source and the fluid flow of the pressure source. For instance, if a pressure source with higher pressure and/or larger fluid flow is used the characteristic peak may occur sooner, such as after 0.1 seconds or 50 milliseconds.

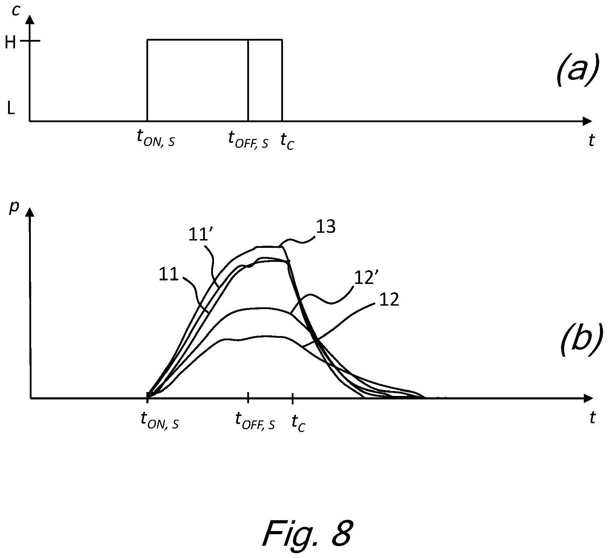

With reference to fig. 8a a short period of time or short pulse is introduced. The short period of time or short pulse is e.g. sufficiently short such that the characteristic peak is not reached. For instance, the short predetermined period of time is between 1 millisecond and 300 milliseconds, such as 2, 5, 10, 100 or 200 milliseconds. The control signal c for the short period of time is at its high level between t_{ON}, s and t_{OFF}, s during which time a fluid is injected to the first lumen 120 from the pressure source 160.

Fig. 8b illustrates the pressure in the first lumen 120 as a function of time during the short period of time for a catheter with a new inflatable retention element of a first size (pressure function 11), a catheter with a new inflatable retention element of a second size (pressure function 11'), a catheter with a used inflatable retention element of the first size (pressure function 12), a catheter with a used inflatable retention element of the second size (pressure function 12') and a catheter without an inflatable retention element (pressure function 13), wherein the second size is smaller than the first size. A similar distribution of curves is obtained for the current or voltage supplied to the pump for the short period of time.

Accordingly, with sufficient measurement accuracy of the pressure parameter the functional parameters of the catheter 200 may be distinguished by measuring the pressure parameter during the predetermined short period of time. For instance, the rate of change of the pressure curves 11, 11', 12, 12', 13 at the onset or the maximum value reached during the short period of time may be used to determine the functional parameter of the catheter.

Fig. 8a and 8b also illustrates a predetermined resting time between time t_{OFF}, s and time t_{C}. At t_{OFF}, s the pressure source is inactivated but the venting valve 175 valve closed while at tc the venting valve 175 is opened to venting the first lumen 120. Accordingly, the pressure functions 11, 11', 12, 12', 13 remain approximately constant during the predetermined resting time whereas the pressure starts to decrease after tc when the venting valve 175 is opened.

A similar resting time may be established for the long predetermined period of time described in fig. 5a by inactivating the pressure source some time before the valve is closed and the venting valve 175 is opened. The pressure parameter may be measured at any time during at least one of the predetermined periods of time when the valve is open and/or the pressure source is active, the resting period of time when the pressure source is inactivated or the second period of time during which the first lumen is vented.

The person skilled in the art realizes that the present invention by no means is limited to the preferred embodiments described above. On the contrary, many modifications and variations are possible within the scope of the appended claims. For example, the sensor may measure both the fluid pressure in the first lumen and the current or voltage supplied to the pump wherein the functional parameter is based on at least two pressure parameters, namely the measured pressure in the first lumen and the current or voltage supplied to the pump.

## Claims

1. A method for determining a functional parameter of a catheter, the method comprising:
connecting said catheter to a catheter connector interface, said catheter connector interface comprising a first lumen configured to be in fluid communication with an inflatable retention element of the catheter and a second lumen configured to be in fluid communication with a main lumen of the catheter;
injecting a fluid into the first lumen during a predetermined period of time;
measuring a pressure parameter related to the pressure in the first lumen; and
determining, based on the measured pressure parameter, a functional parameter of the catheter.

2. The method according to claim 1, wherein measuring a pressure parameter related to the pressure in the first lumen further comprises:
measuring a fluid pressure in the first lumen with a pressure sensor;
wherein the pressure parameter is based on the fluid pressure.

3. The method according to claim 1 or 2, wherein injecting a fluid into the first lumen comprises:
operating an electrical pump to pump said fluid into the first lumen;
wherein the pressure parameter is determined based on a voltage or a current supplied to the pump.

4. The method according to any of the preceding claims, wherein determining a functional parameter comprises:
determining if the pressure parameter is above a first predetermined threshold;
if the pressure parameter is above the first predetermined threshold, assigning the functional parameter with a first value; and else
assigning the functional parameter with a second value.

5. The method according to claim 4, wherein determining a functional parameter further comprises:
determining if the pressure parameter is above a second predetermined threshold value, the second predetermined threshold value being higher than said first predetermined threshold value; and
if the pressure parameter is above the second predetermined threshold, assigning the functional parameter with a third value.

6. The method according to claim 4 or 5, wherein the first value of the functional parameter indicates that the catheter comprises a new inflatable retention element and wherein the second value of the functional parameter indicates that the catheter comprises a used inflatable retention element.

7. The method according to claim 5, wherein the third value of the functional parameter indicates that the first lumen is disconnected from fluid communication with an inflatable retention element.

8. The method according to any of the preceding claims wherein measuring a pressure parameter related to the pressure in the first lumen comprises:
measuring a plurality of pressure parameter samples, wherein the pressure parameter is based on said plurality of pressure parameters samples.

9. The method according to any of the preceding claims, wherein the first lumen is separated from a pressure source with a valve, the method further comprising:
opening said valve for the predetermined period of time to inject a pressurized fluid from the pressure source into the first lumen during the predetermined period of time.

10. The method according to any of the preceding claims, wherein measuring the pressure parameter comprises measuring the pressure parameter during the predetermined period of time.

11. The method according any of the preceding claims, wherein said predetermined period of time is a first period of time, wherein the method further comprises:
ceasing injection of the fluid after said predetermined first period of time; and wherein
measuring the pressure parameter comprises measuring the pressure parameter during a second period of time, the second period of time being subsequent to said first period of time.

12. The method according to any of the preceding claims, wherein said functional parameter is indicative of at least one of: whether the catheter comprises an inflatable retention element, whether the inflatable retention element is new or used, and a size of the inflatable retention element.

13. The method according to any of the preceding claims wherein said predetermined period of time is between 1 millisecond and 10 seconds and preferably between 3 milliseconds and 5 seconds.

14. A catheter control assembly comprising:
a catheter connector interface, comprising:
a first lumen configured to be in fluid communication with an inflatable retention element of the catheter,
a second lumen configured to be in fluid communication with a main lumen of a catheter, and
a catheter controlling unit, the catheter controlling unit being configured to
operate fluid injection means to inject a fluid into the first lumen during a predetermined period of time,
measure a pressure parameter related to the pressure in the first lumen, and
determine, based on the measured pressure parameter, a functional parameter of the catheter.

15. A computer program product comprising instructions which, when executed by a computer, performs the method according to any of claims 1-13.
